# EUROPEAN PATENT APPLICATION

(11) **EP 4 782 409 A1**
(43) Date of publication of application: **29.07.2026**
(21) Application number: 26153690.8
(22) Date of filing: 23.01.2026
(51) Int. Cl.: C02F 1/467, B63B 35/32, C02F 1/68, C02F 1/76, E02B 15/04, E02B 15/10, C02F 1/40

(54) **WATERBORNE CLEANING ROBOT WITH STERILIZATION FUNCTION**

(30) Priority: 23.01.2025 CN 202520160329 U
(71) Applicant: Ningbo SurgeEdge Management Co., Ltd, Ningbo City Zhejiang Province (CN)
(72) Inventor: CAO, Enguo, Ningbo (CN); XU, Yiwu, Ningbo (CN)
(74) Representative: Pons IP

(57) **Abstract**

The disclosure provides a waterborne cleaning robot with a sterilization function, including a machine body, a storage component, and a sterilization device. The machine body is capable of moving on a water surface. An accommodating cavity is formed in the machine body, and the storage component is detachably mounted in the accommodating cavity. A rolling component capable of bringing garbage into a cavity of the storage component is rotationally mounted on the storage component or the machine body. The sterilization device is configured to sterilize and disinfect water. The sterilization device includes a chlorine tablet disinfection component and/or a electrolytic device, the chlorine tablet disinfection component includes a plurality of chlorine tablets, and at least part of the chlorine tablets are placed in the water. The electrolytic device includes at least one metal electrode, and the metal electrode is partially or completely submerged in the water.

## Description

### Technical Field

The disclosure relates to the field of waterborne cleaning robots, and in particular, to a waterborne cleaning robot with a sterilization function.

### Background

With the development of society and people's increasing emphasis on environmental quality, water surface cleaning has become increasingly important. Various waters such as lakes, rivers, and ponds may be affected by pollutants such as garbage, duckweed, and oil stain, which not only affect aesthetics but may also cause damage to aquatic ecosystems.

Therefore, people have designed water surface cleaning robots. Patent number CN211973429U discloses a water surface cleaning machine with a good storage effect, including a machine body capable of moving on a water surface, and a storage component provided with at least one through hole. The storage component is assembled on the machine body in a detachable manner. The machine body is provided with an accommodating cavity that communicates vertically, and the accommodating cavity is provided with an upper opening that allows for the placement of the storage component from top to bottom. The storage component is of a cavity structure with an open upper end. The storage component or the machine body is rotationally connected with a rolling component capable of driving garbage into the cavity structure. The rolling component is provided with a sheet structure capable of generating water flow, and an upper end of the cavity structure adjacent to the rolling component is open. By the above structure, during movement of the machine body, the rolling component can bring garbage into the storage component. Although the structure can achieve the purpose of clearing the garbage on the water surface, the structure has a relatively single function and cannot simultaneously sterilize and disinfect the water, resulting in a poor using effect.

### Summary

### (I) Technical problem to be solved

The problem to be solved by the disclosure is to provide a waterborne cleaning robot with a sterilization function to overcome the shortcoming of relatively single function of existing waterborne cleaning robots.

### (II) Technical solutions

In order to solve the technical problem, the disclosure provides a waterborne cleaning robot with a sterilization function, including:
a machine body capable of moving on a water surface, wherein a bottom of the machine body is provided with an accommodating cavity;
a storage component detachably mounted in the accommodating cavity, wherein a rolling component capable of bringing garbage into a cavity of the storage component is rotationally mounted on the storage component or the machine body; and
a sterilization device mounted in the machine body and configured to sterilize and disinfect water, wherein the sterilization device includes a chlorine tablet disinfection component and/or a electrolytic device, , the chlorine tablet disinfection component includes a plurality of chlorine tablets, and the electrolytic device generator includes at least one metal electrode; and when the machine body is placed on a water surface, at least part of the chlorine tablets are placed in the water, and the metal electrode is partially or completely submerged in the water.

In this solution, the sterilization device may be a chlorine tablet disinfection component, a electrolytic device, or a combination of the chlorine tablet disinfection component and the electrolytic device. When the machine body moves on the water surface, the storage component cooperates with the rolling component to clear the garbage on the water surface, and at the same time, the sterilization device is in contact with the water, so that the chlorine tablet disinfection component and the electrolytic device can sterilize and disinfect the water, thereby increasing the functional diversity of the waterborne cleaning robot, enabling the waterborne cleaning robot to have both garbage clearing and sterilizing and disinfecting functions, and achieving a better using effect.

In some embodiments, the plurality of chlorine tablets are vertically stacked, and the bottommost chlorine tablet can be placed in water; and a front end of the storage component is provided with an input port, the rolling component is located on a front side of the input port, and the rolling component is enabled to directly face the input port to conveniently sweep the garbage on the water surface such as fallen leaves into the storage component through the input port.

In some embodiments, the chlorine tablet disinfection component includes a floating seat slidably arranged along a vertical direction, the floating seat is located on an upper side of the adjustable support, the plurality of chlorine tablets are stacked at an upper end of the floating seat, and the floating seat is capable of enabling at least part of the chlorine tablets located at the bottom to be placed in the water under the gravity of the chlorine tablets, that is, when the floating seat is located at the bottommost position, at least part of the chlorine tablets at the upper end of the floating seat can be placed in water.

In some embodiments, the chlorine tablet disinfection component further includes a chlorine tablet cylinder vertically arranged on the machine body, and the floating seat is slidably mounted inside the chlorine tablet cylinder.

In some embodiments, a height-adjustable support is mounted at a lower end inside the chlorine tablet cylinder; and the floating seat is located on an upper side of the adjustable support, and the floating seat on which the plurality of chlorine tablets are mounted abuts against the adjustable support. The adjustable support is in threaded connection in the chlorine tablet cylinder, the adjustable support is provided with a support plate for supporting the floating seat, and the support plate is provided with a plurality of water passing holes annularly at equal intervals; and the support plate and the floating seat are located below the water surface so that at least part of the chlorine tablets are placed in the water.

In this solution, by arranging the height-adjustable support, according to actual use requirements, the support height of the adjustable support can be adjusted, and the number of chlorine tablets entering the water can be adjusted, thereby adjusting the sterilization effect according to actual situations of water areas, and making the use more flexible; and by arranging the floating seat and stacking the plurality of chlorine tablets at the upper end of the floating seat, after the chlorine tablet placed in the water is completely exhausted, the next chlorine tablet (or next few chlorine tablets) can enter the water again to achieve the function of automatically adding chlorine tablets, so that the degree of automation is high, and the use is more flexible.

In some embodiments, the buoyancy of the floating seat is less than the gravity of a single chlorine tablet, a sensing device is mounted on the floating seat, and when the floating seat rises to the water surface, the sensing device sends a sensing signal for indicating that the chlorine tablets are exhausted; one side or two sides of the floating seat are provided with guide columns, and the chlorine tablet cylinder is provided with guide grooves adapted to the guide columns along a vertical direction; and a cylinder cover is in threaded connection with a top end of the chlorine tablet cylinder.

In some embodiments, the electrolytic device includes a main body bracket arranged on the machine body and an electrode holder detachably connected to the main body bracket, and the metal electrode is vertically mounted on the electrode holder; and an outer wall of the main body bracket is provided with a threaded column, a fastening nut is in threaded connection with the threaded column, and the electrode holder is connected to the main body bracket through the fastening nut. A lower end of the electrode holder is provided with a filter basket around an outer side of the metal electrode, a slag collecting box is detachably mounted at a lower end of the filter basket, and the filter basket is provided with a funnel part at one end of the slag collecting box.

In this solution, the electrolytic device and the machine body are convenient to disassemble and assemble and can be conveniently disassembled for replacing when the electrode is exhausted. By arranging the filter basket cooperating with the slag collecting box, on the premise that the filter basket ensures the circulation of water, impurities generated by the electrode can be prevented from leaking out, the collected impurities can be put into the slag collecting box through the funnel part arranged at the lower end, electrode impurities can be collected, and the using effect is good. The slag collecting box is made of a transparent material to facilitate observation. Arranging the funnel part can effectively ensure that impurities are difficult to escape from the slag collecting box after entering the slag collecting box.

In some embodiments, one side of the bottom of the machine body is provided with a storage component support plate, and the other side of the bottom of the machine body is provided with a connection clamping slot; and one end of the storage component is supported on the storage component support plate, a telescopic clamping head capable of being clamped to the connection clamping slot is mounted at the other end of the storage component, and a bottom of the storage component is provided with a knob for driving the telescopic clamping head to extend and retract.

In some embodiments, the storage component is of a basket-shaped frame structure, the rolling component is rotationally mounted on the machine body, a primary gear is mounted at one end of the rolling component, a drive motor is mounted on the machine body, a secondary gear is mounted on the drive motor, and the secondary gear is in driving connection with the primary gear; and a storage component cover plate is hinged to an upper end of the storage component.

In some embodiments, an electrical control box is mounted on the machine body on one side of the storage component, and the chlorine tablet disinfection component is mounted at a side end of the electrical control box; and the electrolytic device is detachably mounted on one side of the electrical control box, and is electrically connected to the electrical control box.

In some embodiments, a cover plate is mounted on the machine body, and the cover plate covers the sterilization device and the storage component; and a solar panel is mounted on the cover plate.

In some embodiments, the machine body includes floating boxes symmetrically arranged on two sides, and propellers are symmetrically mounted at a rear end of the machine body; and sensing radars for sensing surrounding obstacles are mounted at the front and rear ends and on both sides of the machine body.

In a preferred embodiment the electrolytic device is a metal ion generator.

### (III) Beneficial effects

Compared with the prior art, the waterborne cleaning robot with a sterilization function, provided by the disclosure, has the following advantages:
(1) The sterilization device is additionally arranged, and the sterilization device may be the chlorine tablet disinfection component, the electrolytic device, or a combination of the chlorine tablet disinfection component and the electrolytic device. When the machine body moves, the storage component cooperates with the rolling component to clear the garbage on the water surface, and at the same time, the chlorine tablet disinfection component and the electrolytic device can sterilize and disinfect the water, thereby increasing the functionality of the waterborne cleaning robot, enabling the waterborne cleaning robot to have both garbage clearing and sterilizing and disinfecting functions, and achieving a better using effect.
(2) The chlorine tablet disinfection component can adjust the number of chlorine tablets entering the water and adjust the sterilization effect through the cooperation between the adjustable support and the floating seat. The function of automatically adding chlorine tablets to the water can be achieved, the degree of automation is high, and the use is convenient.
(3) When the electrolytic device is a metal ion generator, a copper ion generator can be used as the metal ion generator and has a good sterilization effect. Furthermore, by arranging the filter basket cooperating with the slag collecting box, on the premise that the filter basket ensures the circulation of water, impurities generated by the electrode can be prevented from leaking out, the collected impurities can be put into the slag collecting box through the funnel part arranged at the lower end, electrode impurities can be collected, and the using effect is good.
(4) The front end of the storage component is provided with the input port, and the rolling component is located on the front side of the input port, thereby facilitating the rolling component to guide the water flow and sweep the garbage on the water surface into the storage component through the input port.

### Brief Description of Figures

In order to illustrate the technical solutions in the embodiments of the disclosure more clearly, the following briefly introduces the accompanying drawings required in the embodiments. Apparently, the accompanying drawings in the following descriptions show only some embodiments of the disclosure, and those of ordinary skill in the art can still derive other drawings according to these accompanying drawings without creative efforts.
FIG. 1 is a three-dimensional view of a waterborne cleaning robot with a sterilization function in the disclosure;
FIG. 2 is a three-dimensional view of another perspective of a waterborne cleaning robot with a sterilization function in the disclosure;
FIG. 3 is an exploded view of a waterborne cleaning robot with a sterilization function in the disclosure;
FIG. 4 is a three-dimensional view of a sterilization device of a waterborne cleaning robot with a sterilization function in the disclosure;
FIG. 5 is a cross-sectional view of a chlorine tablet disinfection component of a waterborne cleaning robot with a sterilization function in the disclosure;
FIG. 6 is an exploded view of a chlorine tablet disinfection component of a waterborne cleaning robot with a sterilization function in the disclosure;
FIG. 7 is a three-dimensional view of an adjustable support of a waterborne cleaning robot with a sterilization function in the disclosure;
FIG. 8 is an exploded view of a filter basket and a slag collecting box of a waterborne cleaning robot with a sterilization function in the disclosure;
FIG. 9 is a cross-sectional view of a electrolytic device of a waterborne cleaning robot with a sterilization function in the disclosure;
FIG. 10 is a three-dimensional view of a storage component of a waterborne cleaning robot with a sterilization function in the disclosure;
FIG. 11 is a three-dimensional view of a machine body of a waterborne cleaning robot with a sterilization function in the disclosure; and
FIG. 12 is a three-dimensional view of a storage component of a waterborne cleaning robot with a sterilization function in the disclosure.

The names of components corresponding to reference numerals in the figures are as follows: 1: machine body; 11: drive motor; 12: secondary gear; 13: floating box; 14: propeller; 101: accommodating cavity; 102: storage component support plate; 103: connection clamping slot; 2: storage component; 21: rolling component; 22: primary gear; 23: storage component cover plate; 24: telescopic clamping head; 25: knob; 201: input port; 3: chlorine tablet disinfection component; 31: chlorine tablet; 32: chlorine tablet cylinder; 33: adjustable support; 34: floating seat; 35: cylinder cover; 321: guide groove; 331: support plate; 332: water passing hole; 341: guide column; 4: electrolytic device; 41: metal electrode; 42: electrode holder; 43: main body bracket; 44: filter basket; 45: slag collecting box; 46: fastening nut; 431: threaded column; 441: funnel part; 5: electrical control box; 6: cover plate; 61: clamping component; 7: solar panel.

### Detailed Description

The disclosure will be described in detail below in conjunction with accompanying drawings and specific embodiments.

The implementations of the disclosure are described below through specific examples. Those skilled in the art can easily understand other advantages and effects of the disclosure from the content disclosed in the specification. It is apparent that the described embodiments are only a part of the embodiments of the disclosure, rather than all of the embodiments. The disclosure may also be implemented or applied through other different specific implementations, and various details in the specification may also be modified or changed based on different viewpoints and applications without departing from the spirit of the disclosure. It should be noted that the following embodiments and the features in the embodiments can be combined with each other in the case of no conflict. All other embodiments obtained by those of ordinary skill in the art based on the embodiments in the disclosure without creative efforts shall fall within the protection scope of the disclosure.

It is noted that various aspects of the embodiments within the scope of the appended claims are described below. It is apparent that the aspects described herein can be embodied in a wide variety of forms, and any specific structure and/or function described herein is merely illustrative. Based on the disclosure, those skilled in the art should understand that one aspect described herein may be implemented independently of any other aspect, and two or more of these aspects may be combined in various ways. For example, an apparatus and/or a method may be implemented using any number of aspects described herein. In addition, the apparatus and/or the method may be implemented using structures and/or functionalities other than one or more of the aspects described herein.

It should also be noted that the figures provided in the following embodiments merely illustrate the basic concept of the disclosure by way of illustration, and only the components related to the disclosure are shown in the figures and are not drawn according to the number, shape, and size of the components in actual implementation, and the form, number, and proportion of the components in actual implementation may be arbitrarily changed, and the layout of the components may be more complicated.

In addition, in the following description, specific details are provided to facilitate a thorough understanding of the examples. However, it will be understood by those skilled in the art that the disclosure may be implemented without these specific details.

The technical solutions provided in the embodiments of the disclosure will be described in conjunction with the accompanying drawings.

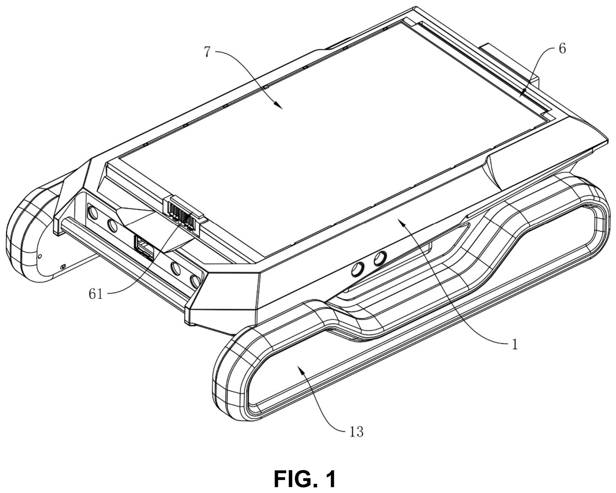

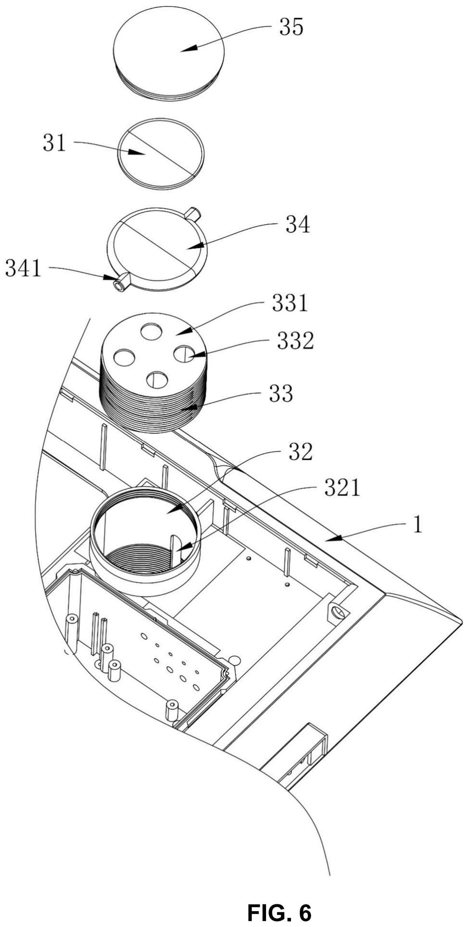

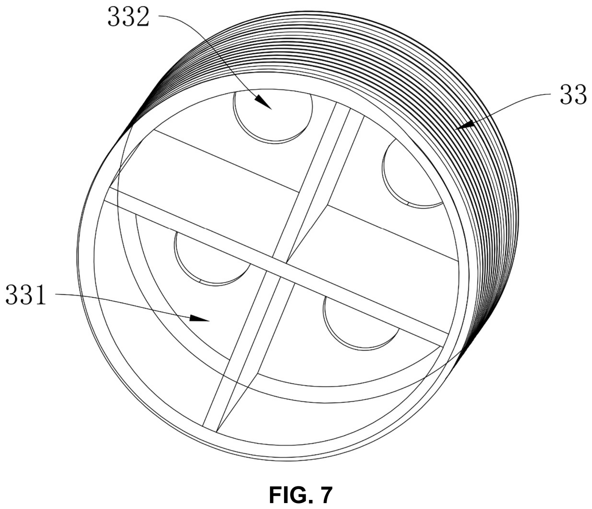

Referring to FIG. 1 to FIG. 11, the disclosure provides a waterborne cleaning robot with a sterilization function, including a machine body 1, a storage component 2, and a sterilization device.

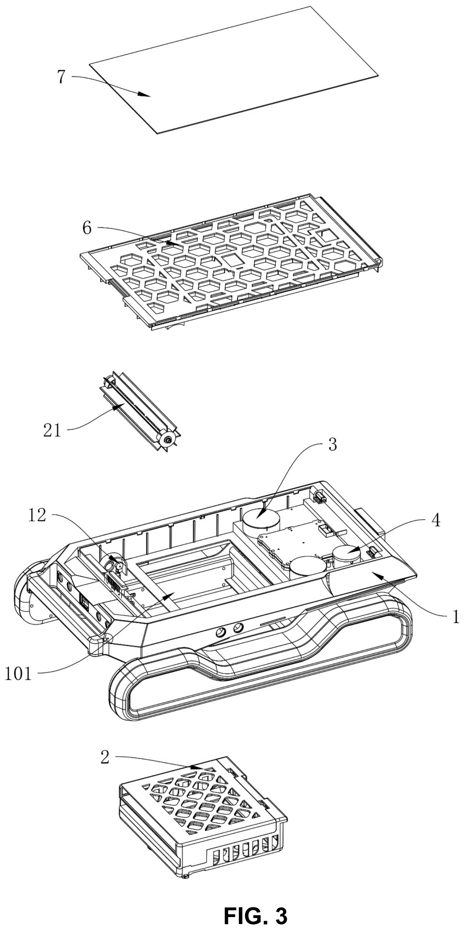

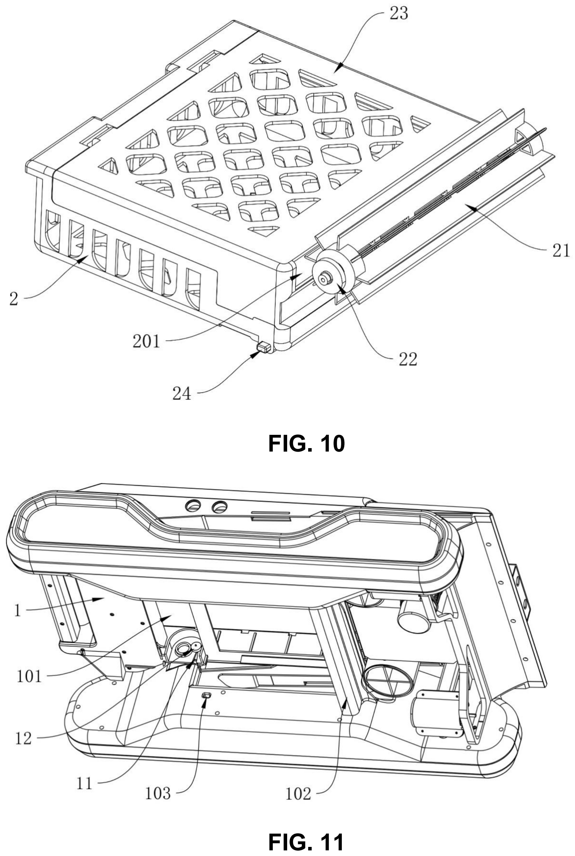

The machine body 1 is capable of moving on a water surface. Referring to FIG. 3, a bottom of the machine body 1 is provided with an accommodating cavity 101, and the accommodating cavity 101 is configured to carry the storage component 2. The storage component 2 is configured to collect and accommodate garbage on the water surface, the storage component 2 is detachably mounted in the accommodating cavity 101 at the bottom of the machine body 1, and the storage component 2 may be mounted from the bottom of the machine body 1 to the machine body 1. A rolling component 21 capable of bringing garbage into a cavity of the storage component 2 is rotationally mounted on the storage component 2 or the machine body 1. As shown in FIG. 10, in this embodiment, the rolling component 21 is rotationally mounted on the machine body 1, the rolling component 21 is separated from the storage component 2, and the rolling component 21 has a sheet structure capable of generating water flow to enable the garbage on the water surface to enter the cavity of the storage component 2 along the water flow.

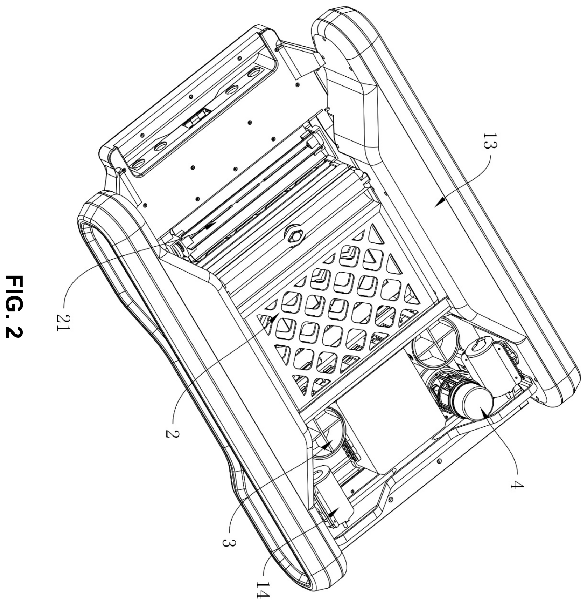

Referring to FIG. 2 to FIG. 5 and FIG. 8, the sterilization device is mounted at a side end of the machine body 1 located in the accommodating cavity 101. The sterilization device is configured to sterilize and disinfect the water, the use functionality of the device is increased, and thus, the device has both garbage clearing and sterilizing and disinfecting functions and has stronger functionality. The sterilization device includes a chlorine tablet disinfection component 3 and/or a electrolytic device 4, that is, the sterilization device may use the chlorine tablet disinfection component 3 or the
electrolytic device 4, or a combination of the chlorine tablet disinfection component 3 and the electrolytic device 4. In this embodiment, in order to ensure sterilizing and disinfecting effects, the sterilization device uses the combination of the chlorine tablet disinfection component 3 and the electrolytic device 4. The sterilization device may be selected according to needs during actual use.

The chlorine tablet disinfection component 3 includes a plurality of chlorine tablets 31 stacked along a vertical direction, and each of the chlorine tablets 31 is in a circular sheet shape. When the machine body 1 is placed on the water surface, at least part of the chlorine tablets 31 located at the bottom are placed in the water. A copper ion generator is used as the electrolytic device 4, and the electrolytic device 4 includes at least one metal electrode 41. In this embodiment, two metal electrodes 41 arranged oppositely at an interval are used. The number of metal electrodes 41 may be set according to actual needs. When the machine body 1 is placed on the water surface, the metal electrode 41 is partially or completely submerged in the water. According to sterilization needs, the bottommost chlorine tablet 31 may be partially or completely placed in the water, or several bottom chlorine tablets 31 may be placed in the water.

When the machine body moves, the storage component cooperates with the rolling component to clear the garbage on the water surface, and at the same time, the chlorine tablet disinfection component and the electrolytic device can sterilize and disinfect the water, thereby increasing the functionality of the waterborne cleaning robot, enabling the waterborne cleaning robot to have both garbage clearing and sterilizing and disinfecting functions, and achieving a better using effect. It should be noted that preferably, the metal electrode 41 is completely submerged in the water, that is, when the machine body 1 is placed on the water surface, the metal electrode 41 is located below the water surface.

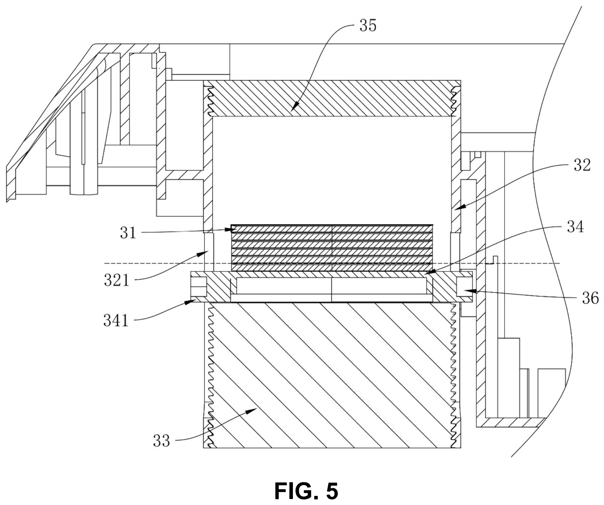

In some embodiments, as shown in FIG. 5 to FIG. 7, the chlorine tablet disinfection component 3 includes a floating seat 34 slidably arranged along a vertical direction, the plurality of chlorine tablets 31 are stacked at an upper end of the floating seat 34, and the floating seat 34 is capable of enabling at least part of the chlorine tablets 31 located at the bottom to be placed in the water under the gravity of the chlorine tablets 31. Further, the chlorine tablet disinfection component 3 also includes a chlorine tablet cylinder 32 vertically arranged on the machine body 1, a through cavity for accommodating the plurality of chlorine tablets 31 is formed in the chlorine tablet cylinder 32, and the chlorine tablet cylinder 32 and the machine body 1 are integrated. The floating seat 34 is slidably mounted inside the chlorine tablet cylinder 32. The buoyancy of the floating seat 34 is less than the gravity of a single chlorine tablet 31. The floating seat 34 with the chlorine tablets 31 will be placed in the water. After the chlorine tablets 31 are completely consumed, the floating seat 34 will float up again.

In some embodiments, as shown in FIG. 5 to FIG. 7, a height-adjustable support 33 is mounted at a lower end inside the chlorine tablet cylinder 32, the floating seat 34 is located on an upper side of the adjustable support 33, and the floating seat 34 on which the plurality of chlorine tablets 31 are mounted will abut against the adjustable support 33, that is, the adjustable support 33 defines the lowest position of the floating seat 34. The adjustable support 33 is configured to adjust the number of the chlorine tablets 31 entering the water, that is, to adjust the draft of the chlorine tablets 31.

By arranging the height-adjustable support 33, according to actual use requirements, the support height of the adjustable support 33 can be adjusted, that is, the number of chlorine tablets entering the water can be adjusted, thereby adjusting the sterilization effect according to actual situations of water areas, and making the use more flexible. The floating seat 34 is arranged, the buoyancy of the floating seat 34 is less than the gravity of a single chlorine tablet 31, the chlorine tablets 31 are placed on the floating seat 34, and the bottommost chlorine tablet 31 is placed in the water. After the chlorine tablet 31 placed in the water is completely melted, the chlorine tablet 31 at the upper layer enters the water again until all chlorine tablets 31 are consumed. The chlorine tablet disinfection component 3 can achieve the function of automatically adding chlorine tablets, the degree of automation is high, and the use is more flexible.

In some embodiments, as shown in FIG. 5 to FIG. 7, the adjustable support 33 is in threaded connection with the chlorine tablet cylinder 32. During height adjustment, the adjustable support 33 can be rotated directly, so that the operation is simple and convenient. The adjustable support 33 is provided with a support plate 331 for supporting the floating seat 34, the support plate 331 is provided with a plurality of water passing holes 332 annularly at equal intervals, and the water passing holes 332 can ensure that the chlorine tablets located at the bottom are placed in the water. The support plate 331 and the floating seat 34 are located below the water surface so that at least part of the chlorine tablets 31 are placed in the water.

In some embodiments, as shown in FIG. 5 to FIG. 7, a sensing device is mounted on the floating seat 34, the sensing device includes a magnet component 36 mounted on the floating seat 34, and a sensor that may sense the magnet component 36 is mounted on the machine body 1. In this embodiment, a magnet component can be mounted in the groove of the guide column 341. When all chlorine tablets are exhausted, the floating seat 34 floats up to the water surface due to the lack of the pressing force of the chlorine tablets 31, then the magnet component 36 corresponds to the sensor in position and the sensor senses the magnet component 36, and the sensor sends a sensing signal for indicating that the chlorine tablets are exhausted, so that a caution light on the machine body gives an alarm. The sensor belongs to the prior art and will not be described in this embodiment. By additionally arranging the sensor, when all chlorine tablets 31 in the chlorine tablet cylinder 32 are exhausted, an alarm can be given by sensing, and a user can add chlorine tablets 31 in time. One side or two sides of the floating seat 34 are provided with guide columns 341, the chlorine tablet cylinder 32 is provided with guide grooves 321 adapted to the guide columns 341 along a vertical direction, the guide columns 341 are arranged in the guide grooves 321, and the guide effect is good. A cylinder cover 35 is in threaded connection with a top end of the chlorine tablet cylinder 32. The cylinder cover 35 can prevent water from entering from the upper end of the chlorine tablet cylinder 32 to prevent the chlorine tablets 31 that are not placed in water from being wet or damaged, so that the protection effect is good.

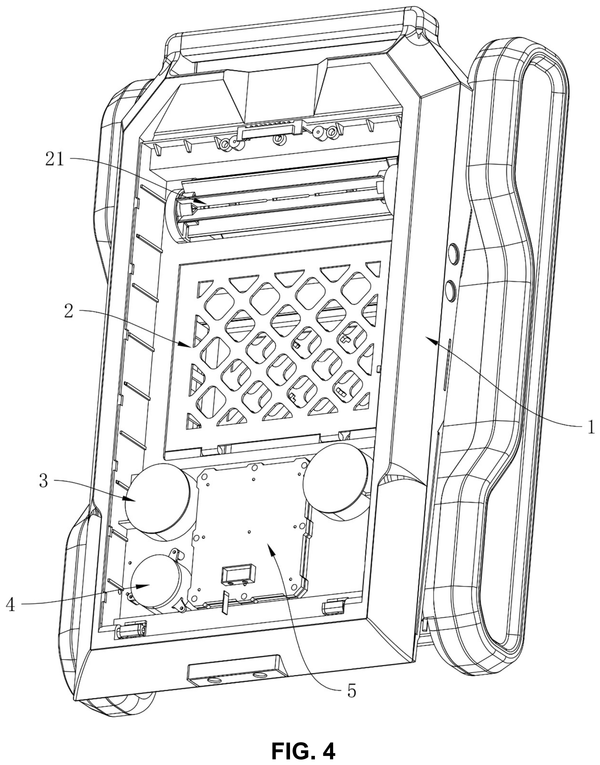

In some embodiments, as shown in FIG. 2 and FIG. 4, an electrical control box 5 is mounted on the machine body 1 at a middle position of one side of the storage component 2, and the electrical control box 5 is electrically connected to the sensor in the chlorine tablet cylinder 32. The chlorine tablet disinfection component 3 is mounted at a side end of the electrical control box 5 to ensure sterilizing and disinfecting effects. Specifically, two groups of chlorine tablet disinfection components 3 are provided and are symmetrically mounted on two sides of the electrical control box 5. The electrolytic device 4 is detachably mounted on one side of the electrical control box 5, and the electrolytic device 4 is electrically connected to the electrical control box 5.

In some embodiments, as shown in FIG. 4, FIG. 8, and FIG. 9, the electrolytic device 4 includes a main body bracket 43 arranged on the machine body 1 and an electrode holder 42 detachably connected to the main body bracket 43, the main body bracket 43 and the machine body 1 may be of an integrated structure, an upper side of the main body bracket 43 is connected to an electrically controlled upper cover, the electrically controlled upper cover is electrically connected to the electrical control box 5, and the metal electrode 41 is vertically mounted on the electrode holder 42.

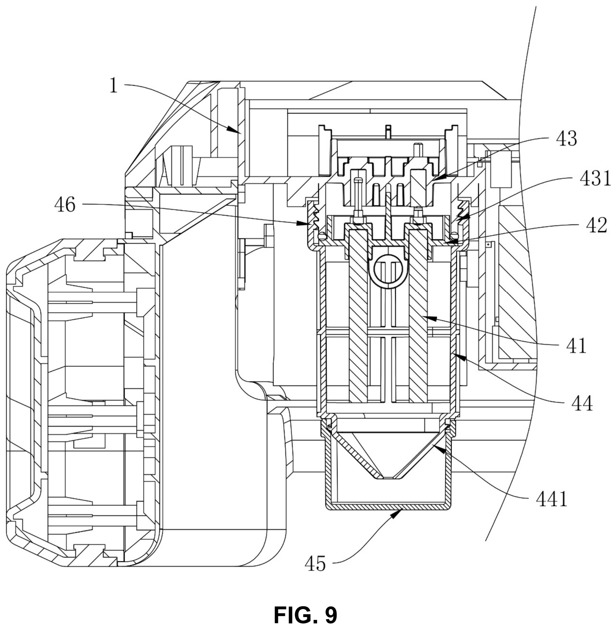

In some embodiments, as shown in FIG. 9, this embodiment provides a specific structure with a detachable electrode holder 42: an outer wall of the main body bracket 43 is provided with a threaded column 431, a fastening nut 46 is in threaded connection with the threaded column 431, and the electrode holder 42 is detachably connected to the main body bracket 43 through the fastening nut 46. Due to this structure, the electrode holder 42 and the main body bracket 43 are convenient to disassemble and assemble. When the electrode is exhausted, the electrode holder and the electrode can be directly disassembled from the bottom of the machine body 1 without the need to open or close the cover plate, thereby saving time and labor in operation and facilitating electrode replacement.

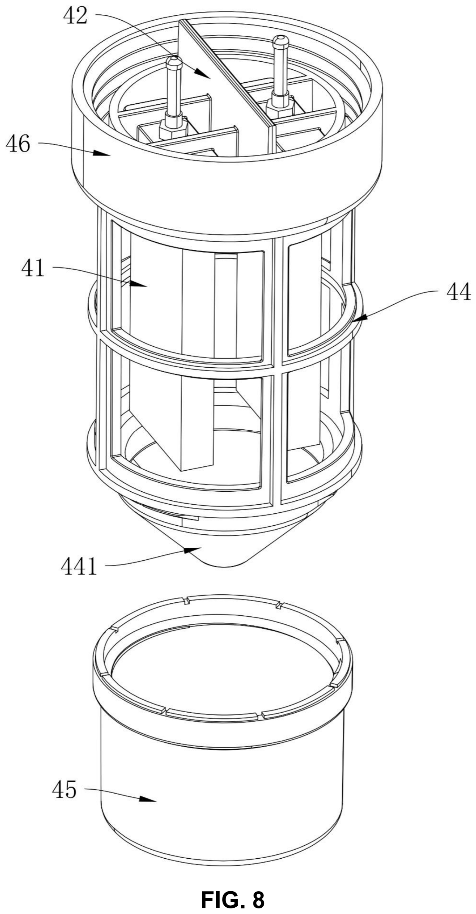

In some embodiments, as shown in FIG. 8 and FIG. 9, a lower end of the electrode holder 42 is provided with a filter basket 44 around an outer side of the metal electrode 41, an outer wall of the filter basket 44 is provided with a filter screen, a slag collecting box 45 is detachably mounted at a lower end of the filter basket 44, and the filter basket 44 is provided with a funnel part 441 at one end of the slag collecting box 45. By arranging the filter basket cooperating with the slag collecting box, on the premise that the filter basket ensures the circulation of water, impurities generated by the electrode can be prevented from leaking out, the collected impurities can be put into the slag collecting box through the funnel part arranged at the lower end, electrode impurities can be collected, and the using effect is good. The slag collecting box 45 is made of a transparent material to facilitate observation. Arranging the funnel part can effectively ensure that impurities are difficult to escape from the slag collecting box after entering the slag collecting box.

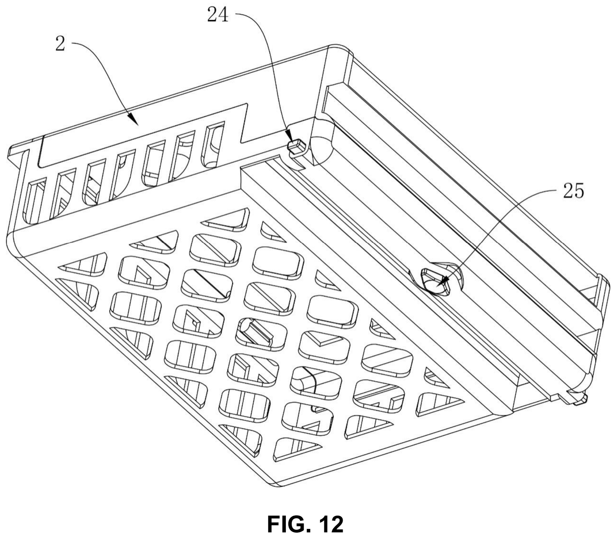

In some embodiments, as shown in FIG. 10 to FIG. 12, one side of the bottom of the machine body 1 is provided with a storage component support plate 102, and the other side of the bottom of the machine body 1 is provided with a connection clamping slot 103 symmetrically. One end of the storage component 2 is supported on the storage component support plate 102, a telescopic clamping head 24 capable of being clamped to the connection clamping slot 103 is mounted at the other end of the storage component 2 symmetrically, and a knob 25 for driving the telescopic clamping head 24 to extend and retract is mounted at the bottom of the storage component 2, thereby achieving detachable connection between the storage component 2 and the machine body 1. The process of driving the telescopic clamping head to extend and retract by the knob 25 belongs to the prior art and will not be described in this embodiment. Due to this structure, the storage component 2 is mounted in a manner of abutting and supporting at one end and clamping at the other end, so that the connection is stable and reliable, and the disassembly and assembly are facilitated. The storage component 2 is of a basket-shaped frame structure. The front end of the storage component 2 is provided with an input port 201, the rolling component 21 is located on a front side of the input port 201, and the rolling component 21 directly faces the input port 201, thereby facilitating the rolling component 21 to guide the water flow and sweep the garbage on the water surface into the storage component 2 through the input port 201.

In an example, as shown in FIG. 3, FIG. 4, and FIG. 11, the rolling component 21 and the storage component 2 are of a split structure, a primary gear 22 is mounted at one end of the rolling component 21, a drive motor 11 is mounted on the machine body 1, a secondary gear 12 is mounted on the drive motor 11, and the secondary gear 12 is in driving connection with the primary gear 22. A storage component cover plate 23 is hinged to an upper end of the storage component 2. By using the storage component cover plate 23 capable of being turned and opened, it is convenient to open the storage component cover plate 23 to clear the garbage inside the storage component 2.

In some embodiments, as shown in FIG. 1 and FIG. 3, a cover plate 6 is mounted at an upper end of the machine body 1, and the cover plate 6 covers the sterilization device and the storage component 2. A solar panel 7 is mounted on the cover plate 6, and the solar panel 7 is electrically connected to the electrical control box 5.

It should be pointed out that the cover plate 6 may be fixedly mounted at the upper end of the machine body 1 through bolts, or may be capable of being opened relative to the machine body 1. As shown in FIG. 1, one end of the cover plate 6 may be hinged to the machine body 1, and the other end of the cover plate 6 is clamped with the machine body 1 through a clamping component 61, so that the cover plate 6 may be opened relative to the machine body 1. The openable cover plate 6 facilitates observation and maintenance.

In some embodiments, as shown in FIG. 1 and FIG. 2, the machine body 1 includes floating boxes 13 symmetrically arranged on two sides, thereby ensuring that the machine body 1 can float on the water surface. Details will not be described in this embodiment. Propellers 14 are symmetrically mounted at a rear end of the machine body 1, and the propellers 14 are configured to enable the machine body 1 to move forward and turn. Sensing radars for sensing surrounding obstacles are mounted at the front and rear ends and on both sides of the machine body 1. The sensing radars are configured to sense surrounding obstacles to facilitate the machine body 1 to avoid obstacles. The sensing radar belongs to the prior art and will not be described in this embodiment.

For the same or similar parts between the embodiments in the specification, reference may be made to each other. Each embodiment focuses on the differences from other embodiments.

The foregoing descriptions are merely specific implementations of the disclosure, but the protection scope of the disclosure is not limited thereto. Any variation or replacement readily figured out by those skilled in the art within the technical scope disclosed in the disclosure shall fall within the protection scope of the disclosure. Therefore, the protection scope of the disclosure shall be subject to the protection scope of the claims.

## Claims

1. A waterborne cleaning robot with a sterilization function, comprising:
a machine body (1) capable of moving on a water surface, wherein a bottom of the machine body (1) is provided with an accommodating cavity (101);
a storage component (2) detachably mounted in the accommodating cavity (101), wherein a rolling component (21) capable of bringing garbage into a cavity of the storage component (2) is rotationally mounted on the storage component (2) or the machine body (1); and
a sterilization device mounted in the machine body (1) and configured to sterilize and disinfect water, wherein the sterilization device comprises a chlorine tablet disinfection component (3) and/or a electrolytic device (4), the chlorine tablet disinfection component (3) comprises a plurality of chlorine tablets (31), and the electrolytic device (4) comprises at least one metal electrode (41); and when the machine body (1) is placed on a water surface, at least part of the chlorine tablets (31) are placed in the water, and the metal electrode (41) is partially or completely submerged in the water.

2. The waterborne cleaning robot with a sterilization function according to claim 1, wherein the plurality of chlorine tablets (31) are vertically stacked; and a front end of the storage component (2) is provided with an input port (201), and the rolling component (21) is located on a front side of the input port (201).

3. The waterborne cleaning robot with a sterilization function according to claim 1 or 2, wherein the chlorine tablet disinfection component (3) comprises a floating seat (34) slidably arranged along a vertical direction, the plurality of chlorine tablets (31) are stacked at an upper end of the floating seat (34), and the floating seat (34) is capable of enabling at least part of the chlorine tablets (31) located at the bottom to be placed in the water under the gravity of the chlorine tablets (31).

4. The waterborne cleaning robot with a sterilization function according to claim 3, wherein the chlorine tablet disinfection component (3) further comprises a chlorine tablet cylinder (32) vertically arranged on the machine body (1), and the floating seat (34) is slidably mounted inside the chlorine tablet cylinder (32); and/or the buoyancy of the floating seat (34) is less than the gravity of a single chlorine tablet (31).

5. The waterborne cleaning robot with a sterilization function according to claim 4, wherein a height-adjustable support (33) is mounted at a lower end inside the chlorine tablet cylinder (32), the floating seat (34) is located on an upper side of the adjustable support (33), the floating seat (34) on which the plurality of chlorine tablets (31) are mounted abuts against the adjustable support (33), and the adjustable support (33) is configured to adjust the number of the chlorine tablets (31) entering the water.

6. The waterborne cleaning robot with a sterilization function according to claim 5, wherein the adjustable support (33) is in threaded connection in the chlorine tablet cylinder (32), the adjustable support (33) is provided with a support plate (331) for supporting the floating seat (34), and the support plate (331) is provided with a plurality of water passing holes (332) annularly at equal intervals; and the support plate (331) and the floating seat (34) are located below the water surface so that at least part of the chlorine tablets (31) are placed in the water.

7. The waterborne cleaning robot with a sterilization function according to claim 3, wherein a sensing device is mounted on the floating seat (34), and when the floating seat (34) rises to the water surface, the sensing device sends a sensing signal for indicating that the chlorine tablets are exhausted; and/or
one side or two sides of the floating seat (34) are provided with guide columns (341), and the chlorine tablet cylinder (32) is provided with guide grooves (321) adapted to the guide columns (341) along a vertical direction.

8. The waterborne cleaning robot with a sterilization function according to claim 1 or 2, wherein the electrolytic device (4) comprises a main body bracket (43) arranged on the machine body (1) and an electrode holder (42) detachably connected to the main body bracket (43), and the metal electrode (41) is vertically mounted on the electrode holder (42).

9. The waterborne cleaning robot with a sterilization function according to claim 8, wherein an outer wall of the main body bracket (43) is provided with a threaded column (431), a fastening nut (46) is in threaded connection with the threaded column (431), and the electrode holder (42) is connected to the main body bracket (43) through the fastening nut (46); and/or a lower end of the electrode holder (42) is provided with a filter basket (44) around an outer side of the metal electrode (41), a slag collecting box (45) is detachably mounted at a lower end of the filter basket (44), and the filter basket (44) is provided with a funnel part (441) at one end of the slag collecting box (45).

10. The waterborne cleaning robot with a sterilization function according to claim 1 or 2, wherein one side of the bottom of the machine body (1) is provided with a storage component support plate (102), and the other side of the bottom of the machine body (1) is provided with a connection clamping slot (103); and one end of the storage component (2) is supported on the storage component support plate (102), a telescopic clamping head (24) capable of being correspondingly clamped to the connection clamping slot (103) is mounted at the other end of the storage component (2), and a bottom of the storage component (2) is provided with a knob (25) for driving the telescopic clamping head (24) to extend and retract.

11. The waterborne cleaning robot with a sterilization function according to claim 1 or 10, wherein the rolling component (21) is rotationally mounted on the machine body (1), a primary gear (22) is mounted at one end of the rolling component (21), a drive motor (11) is mounted on the machine body (1), a secondary gear (12) is mounted on the drive motor (11), and the secondary gear (12) is in driving connection with the primary gear (22); and a storage component cover plate (23) is hinged to an upper end of the storage component (2).

12. The waterborne cleaning robot with a sterilization function according to claim 1, wherein a cover plate (6) is mounted on the machine body (1), and the cover plate (6) covers the sterilization device and the storage component (2); a solar panel (7) is mounted on the cover plate (6); and/or
the machine body (1) comprises floating boxes (13) symmetrically arranged on two sides, and propellers (14) are symmetrically mounted at a rear end of the machine body (1); and/or an electrical control box (5) is mounted on the machine body (1) on one side of the storage component (2), and the chlorine tablet disinfection component (3) is mounted at a side end of the electrical control box (5); and the electrolytic device (4) is detachably mounted on one side of the electrical control box (5), and is electrically connected to the electrical control box (5).

13. The waterborne cleaning robot with a sterilization function according to any of claims 1 to 12 wherein electrolytic device (4) is a metal ion generator.
